# EUROPEAN PATENT SPECIFICATION

(11) **EP 3 554 483 B1**
(45) Date of publication and mention of the grant of the patent: **31.01.2024**
(21) Application number: 17825457.9
(22) Date of filing: 14.12.2017
(51) Int. Cl.: A61K 31/00, A61K 9/00, A61K 31/21, A61K 31/34, A61K 31/7024, A61P 9/00, A61P 9/10

(54) **REDUCING SIDE EFFECTS OF SHORT ACTING NO DONORS**
VERMINDERUNG DER NEBENWIRKUNGEN VON KURZZEITIG WIRKENDEN NO-DONOREN
RÉDUCTION DES EFFETS SECONDAIRES DES DONNEURS DE NO À COURTE DURÉE D'ACTION

(30) Priority: 14.12.2016 EP 16204170
(43) Date of publication of application: 23.10.2019
(73) Proprietor: G. Pohl-Boskamp GmbH & Co. KG, 25551 Hohenlockstedt (DE)
(72) Inventor: BOSKAMP, Marianne, 25524 Bekmünde (DE)
(74) Representative: Simmons & Simmons LLP (Munich)
(86) International application number: PCT/EP2017/082932
(87) International publication number: WO 2018/109131

(56) References cited:
- EP-A1- 2 668 947
- EP-A1- 2 805 730
- Anonymous: "Isosorbide mononitrate 40mg Tablets - (eMC)", , 9 September 2014 (2014-09-09), XP055377173, Retrieved from the Internet: URL:https://www.medicines.org.uk/emc/print -document?documentId=25948 [retrieved on 2017-05-30]
- Anonymous: "Imdur Tablets 60mg - (eMC) print friendly", , 26 May 2015 (2015-05-26), XP055377238, Retrieved from the Internet: URL:https://www.medicines.org.uk/emc/print -document?documentId=178 [retrieved on 2017-05-30]
- Anonymous: "Isosorbide mononitrate 40mg Tablets - Summary of Product Characteristics (SPC) - (eMC)", , 9 September 2014 (2014-09-09), XP055377155, Retrieved from the Internet: URL:https://www.medicines.org.uk/emc/medic ine/25948 [retrieved on 2017-05-30]
- M. Rapado-Castro ET AL: "Cognitive effects of adjunctive N-acetyl cysteine in psychosis", PSYCHOLOGICAL MEDICINE, vol. 47, no. 5, 29 November 2016 (2016-11-29), pages 866-876, XP055685745, GB ISSN: 0033-2917, DOI: 10.1017/S0033291716002932
- Müller-Oerlinghausen et al.: "Handbuch der unerwünschten Arzneimittelwirkungen", 31 December 1999 (1999-12-31), Urban & Fischer, München, Jena pages 262-263,

## Description

The present invention relates to nitroglycerin (GTN) for use in methods for reducing side effects of a treatment with nitroglycerin (GTN), wherein said treatment is for the treatment or prevention of an arterial insufficiency, comprising administering during a defined time period a gradually increasing amount of nitroglycerin (GTN) at any dose from 0.05 mg to 2.0 mg in an intermitting manner, wherein the defined time period is 3 days to 40 weeks long.

Short acting NO donors are a well-known treatment of different diseases having their most frequent use in the cardiovascular field.

Cardiovascular diseases as well as other diseases involving a cardiovascular and, more specifically, arterial insufficiency affect a rising patient population, head international mortality and morbidity statistics and have an enormous economic importance. In Germany, for example, about 280.000 patients suffer every year from a cardiac infarct, while about 65.000 patients die.

One important reason for a cardiovascular disease is the partial or complete occlusion of arterial vessels resulting in a reduced supply of oxygen and nutrients of the tissue supplied by the arterial vessel.

Angina pectoris, the medical term for chest pain or discomfort, is a clinical syndrome reflecting inadequate oxygen supply for myocardial metabolic demands with resultant ischemia and is generally caused by obstruction (stenosis), spasm, endothelial or microvascular dysfunction of coronary arteries. Thus, angina pectoris is a symptom of an underlying heart problem, usually coronary artery disease (CAD).

(Short acting) NO donors, and especially nitroglycerin (glyceryl trinitrate, GTN), are used since decades as vasodilating agents in the above mentioned cardiovascular setting for the symptomatic treatment of cardiovascular diseases like coronary artery disease (also referred to as ischemic heart disease (IHD) or coronary heart disease (CHD)), which is the leading cause of death and disability worldwide (McGrae McDermott M., Journal of the American Medical Association, 2007, 297 (11): 1253-1255).

NO donors have been and still are used to treat the symptoms of these diseases without addressing the basic cause, i.e. the etiology, of the underlying disease due to their vasodilating effect on veins and arteries, resulting in a reduced workload and energy consumption of the heart (by decreasing preload and afterload) as well as an increased myocardial oxygen supply (by dilating the coronary arteries). These symptoms include chest pain, pressure, discomfort, or dyspnea.

Due to its short half-life time in blood plasma nitroglycerin is regarded as a short acting NO donor. Short acting NO donors, especially nitroglycerin, have been and are primarily used for the acute relief or acute prophylaxis of angina pectoris attacks, the most common symptom of CAD (Fox K. et al., Guidelines on the management of stable angina pectoris: full text. The Task Force on the Management of Stable Angina Pectoris of the European Society of Cardiology. European Heart Journal doi:10.1093/eurheartj/ehl002; Gibbons R. J. et al., ACC/AHA/ACP-ASIM Guidelines for the Management of Patients With Chronic Stable Angina. Journal of the American College of Cardiology, 1999, 33 (7): 2092-2197).

Unfortunately, the medical use of the pharmaceutically important NO donors produces frequent side effects in a dose-related manner. This is very well known for the use of the NO donors and organic nitrates nitroglycerin and isosorbide dinitrate (ISDN). They may cause side effects like nitrate headache, blood pressure drop, nausea. When used as a rescue medication in case of an acute ischemic event like, for example, an angina pectoris attack due to coronary artery disease, the side effects are tolerated by most of the patients due to the threatening situation of the acute event.

When used as an acute rescue medication, short acting NO donors are administered in a way that they show a rapid onset and short duration of action with regard to the duration of their pharmacological effect, i.e. their vasodilating action. Examples are intravenously administered GTN or sublingually administered GTN or ISDN formulated in immediate-release dosage forms. When administered in this manner both NO donors reach their peak blood plasma levels within minutes after the administration resulting in the fast onset of action.

When NO donors are used prophylactically as a long-term treatment, i.e. for the long-term prophylaxis of angina pectoris attacks, the side effects are known to be most prominent at the beginning of the therapy but subside during a period of days to weeks when the patient ('s) (body) gets used to the treatment.

For such long-term NO treatment, the NO donors have to be administered in a way that that elevated plasma or tissue levels of the NO donor in a therapeutically sufficient amount are generated in the subject resulting in a long duration of vasodilative action. To keep NO donor levels for a long period of time on a high, elevated level in the subject, short acting NO donors have either to be administered continuously (e.g. by continuous infusion of GTN) or by using an extended-release dosage form (e.g. such as a transdermal GTN patch with a controlled release of GTN from a reservoir) or oral retard preparations (e.g. oral ISDN retard). Alternatively, the patient is to be switched to long acting NO donors (e.g. oral isosorbide mononitrate (ISMN)) that have a long half-life time in the subject's body after their administration and are therefore long acting resulting in a prolonged antianginal activity.

The halftime of isosorbide mononitrate after oral administration is around 5 hours.

In EP2805730, it is described the treatment of chronic fatigue syndrome using a nitric oxide donor.

In WO 2013/178715, it is disclosed that short acting NO donors are able to treat and/or to prevent arterial insufficiencies via the induction of arteriogenesis when administered in an intermitting manner.

This implicates that short acting donors are used in a new and different way other than in the emergency setting described so far. In this case, patients are not in a threatening situation and will therefore more likely refuse to accept additional side effects caused by such a therapy.

Arteriogenesis is a process in which already pre-existing small arteriolar collaterals can develop to full functional conductance arteries which bypass the site of an arterial occlusion and/or compensate blood flow to ischemic territories supplied by the insufficient artery. Consequently, arteriogenesis is a highly effective endogenous mechanism for the maintenance and regeneration of the blood flow after an acute or chronic occlusive event in an arterial vessel. In this case, the collaterals can function as natural bypasses.

Arteriogenesis is a process distinct from angiogenesis or neovascularization, where a de-novo formation of arterial vessels occur (Buschmann I. and Schaper W., Journal of Pathology, 2000, 190: 338-342).

Thus, in contrast to the acute symptomatic treatment of cardiovascular diseases due to the vasodilating effect, short acting NO donors can be used intermittently to specifically treat or prevent diseases, i.e. arterial insufficiencies, via the induction of arteriogenesis. The latter addresses the basic cause of the disease, i.e. the insufficient blood flow to ischemic territories supplied by the insufficient artery, by means of augmentation of collateral blood flow.

Consequently, short acting NO donors represent useful medicaments for both the symptomatic treatment of cardiovascular diseases in an emergency setting, i.e. as a rescue medication for the short-term relief or prophylaxis of acute symptoms of the diseases, as well as for the long term prevention or treatment of arterial insufficiencies, such as e.g. cardiovascular diseases.

However, a treatment with short acting NO donors, as described above, is often impaired by the fact that their administration is accompanied by diverse side effects which may severely influence the compliance of the patients.

During the therapy with short acting NO donors such as nitroglycerin or ISDN, the following side effects have been monitored: headache is a very common (10% or more) side effect which is reported in up to 64% of the cases when short acting NO donors are administered for therapeutical means. Common (1% to 10%) side effects are dizziness, lightheadedness, syncope, vertigo and drowsiness. Rarely (0.1% to 0.01%) seen side effects are severe and prolonged headache. Very rare side effects (less than 0.01%) are in form of cerebral ischemia, other side effects that have been reported but without a particular frequency are faintness and somnolence.

Side effects can occur more frequently or severely in special populations. For example, hypotension or dizziness are more likely to occur in small, lightweight persons. Also, populations with different comorbidities may show a higher frequency of side effects.

The above mentioned side effects are known to negatively influence the patient's compliance especially in a situation which is not a medical emergency. When short acting NO donors are administered in an intermittent manner, e.g. for the induction of arteriogenesis, the side effects will therefore refrain patients from using short acting NO donors or will result in a reduced or insufficient compliance.

In view of the above, there is a need for methods for reducing the side effects of short acting NO donors especially when these NO donors are used in form of a long term intermittent treatment in a non-emergency setting.

The invention is defined by the claims. Any subject-matter falling outside the scope of the claims is provided for information purposes only. Any references in the description to methods of treatment refer to the pharmaceutical composition and medicament according to the present invention for use in a method for treatment of the human or animal body by therapy.

In the context of the present invention, and as outlined in the examples, it has surprisingly been shown that the administration of a gradually increasing amount of the short acting NO donor nitroglycerin (GTN) in an intermitting manner results in the reduction of side effects.

The intermittent administration of short acting NO donors, as referred to in the context of the present invention, is used to create repetitive short-lasting episodes of high NO donor or NO blood plasma or tissue levels. On the one hand, the intermittent administration of short acting NO donors has the improved effect that it mimics the physiological situation of the organism as, for example, comparable to the endogenous release of NO upon physical training. On the other hand, the intermittent administration of a short acting NO donor avoids that the subject is developing tolerances against the NO donor and that the subject is developing endothelial dysfunction. The induction of endothelial dysfunction is a parameter which has a prognostic significance in patients with coronary artery disease. The development of tolerances and the induction of endothelial dysfunction are well known disadvantages caused by the sustained, long term exposure to NO donors (Uxa A. et al., Journal of Cardiovascular Pharmacology, 2010, 56 (4): 354-359).

In a first aspect, the present invention relates to nitroglycerin (GTN) for use in a method of reducing side effects of a treatment with nitroglycerin (GTN), wherein said treatment is for the treatment or prevention of an arterial insufficiency, comprising administering during a defined time period a gradually increasing amount of nitroglycerin (GTN) at any dose from 0.05 mg to 2.0 mg in an intermitting manner, wherein the defined time period is 3 days to 40 weeks long.

As used herein, the term "NO donor" refers to either nitric oxide itself or to any molecule which is capable of releasing nitric oxide (NO) after having been administered to a subject.

As used herein, the term "short acting NO donor" refers to a NO donor with a halftime of less than 60, preferably with a halftime of less than 45, more preferably with a halftime of less than 30 and most preferably with a halftime of 15 minutes or less after having been administered to a subject.

It is to be understood that, in the context of the present invention, the term "halftime" refers to the half-life and/or to the half-life time of the NO donor in the subject's body, in particular in the subject's blood plasma.

The short acting NO donor for use according to the present invention is nitroglycerin.

In the case of nitroglycerin, the halftime and its persistence in the body of the subject has been intensively studied, see e.g. Armstrong et al. Circulation 59:585-588 (1979) or Armstrong et al. Circulation 62:160-166 (1980). In general, the halftime of nitroglycerin is 2 to 5 minutes.

As used herein, the term "administration of an NO donor" means that a given dosage of the NO donor is administered. Depending on the way of administration, the skilled person will appreciate that the administration may take some time. Preferably, the NO donor is administered in form of a spray, sprayable or injectable solution, chewable capsule, inhalable gas, inhalable aerosol or powder, granules, powder or a tablet, preferably a sublingual, buccal or chewable tablet, which means that the administration may be completed within seconds. Modes of administration of the NO donor are further discussed below.

According to the invention, nitroglycerin is administered in an intermitting manner.

In the context of the present invention, the term "intermitting manner" means that the NO donor, i.e. nitroglycerin, is administered in a way that its plasma or tissue levels are only elevated in a short-term manner after the NO donor has been administered but then again decline. This can be achieved, for example, if the administration of the short acting NO donor is followed by a time period without administration and then the NO donor is again administered to the subject. Moreover, the administration of nitroglycerin in an intermitting manner according to the present invention has the effect that it mimics the physiological situation of the organism as, for example, comparable to the endogenous release of NO upon physical training. In other words, nitroglycerin acts as a biomimetic when administered to a subject in an intermitting manner.

In a preferred embodiment, the plasma levels of nitroglycerin are elevated for not more than 60 minutes, or for not more than 50, 40, 30, 15, 10 or 5 minutes.

In the context of the present invention, the term "in an intermitting manner" is to be understood that nitroglycerin is not administered continuously, for example, by means of long term intravenous infusion or with the help of an implanted pump which constantly delivers nitroglycerin to the subject.

In the context of the present invention, the term "in an intermitting manner" is to be understood in that nitroglycerin is not administered in a way that it is released for a prolonged, sustained period of time into the subject. This means that nitroglycerin is not administered in a way that a prolonged bioavailability of a therapeutically sufficient amount of nitroglycerin, respectively, is generated in the subject resulting in a long duration of therapeutic action. Thus, in accordance to the present invention, nitroglycerin is not administered in form of an extended-release preparation resulting inevitably in a prolonged nitroglycerin release into the subject, such as, for example, by means of using a transdermal GTN patch with a controlled release of GTN from a reservoir or by means of swallowing an oral retard preparation with a slow and thus sustained release (e.g. oral ISDN retard).

If nitroglycerin is formulated in a sustained-release formulation, the mode of administration has to ensure that nitroglycerin is not released for a prolonged period of time to the subject. For example, this might be achieved by an only short-term adhesion of sustained-release mucosa-adhesive tablet to the mucosa, e.g. a sustained-release mucosa-adhesive buccal tablet, resulting in a short-term release of nitroglycerin into the subject.

Therefore, in a preferred embodiment, nitroglycerin is formulated in a way that it is released over a short period of time after administration. Thereby, nitroglycerin may be released immediately (i.e. immediate-release formulation) as well as after a delayed period of time (delayed-release formulation) after having been administered to the subject.

In a preferred embodiment, nitroglycerin is formulated in an immediate-release formulation. Examples of immediate-release formulations include, but are not limited to, sublingually administered tablets, powder, granules, films, chewable capsules or solution in form of a spray. Other examples include conventional (i.e. non-coated, non-retard) tablets or capsules for oral administration.

Rather, this term also means that there is an interval between two administrations of nitroglycerin, and that nitroglycerin is given several times, e.g. at least 1, 2, 3, 4, 5, 6, 8, 9, 12 or 16 times a day. Equally preferred is that nitroglycerin is administered at least once a day, preferably up to 2 to 3 times a day, and more preferably up to 4 times as day.

Preferably, nitroglycerin is administered three times a day.

As to the schedule of administration, the skilled person will appreciate that there are many ways to achieve the intermitting administration of the invention. For example it is possible to administer the donor at least once a day or, alternatively, at least three times a week for reducing the side effects.

Preferably, during the defined time period in which the amount of nitroglycerin is gradually increased, nitroglycerin may be administered at least once a day, more preferably twice, three times or four times a day, even more preferably under the proviso that the time period between two administrations of nitroglycerin is at least 1 hour, 2 hours or 4 hours, preferably at least 8 hours, more preferably at least 10 hours to 12 hours.

Although possible, it is not necessary that the time periods between two administrations of nitroglycerin are the same. Rather, it is preferred that these time periods differ, depending on the individual administration schedule.

In a preferred embodiment, nitroglycerin is administered at least on three days a week. However, nitroglycerin may also be administered on 4, 5, 6 or 7 days a week. In an especially preferred embodiment, nitroglycerin is administered at least on 3 or 4 days a week. In equally preferred embodiment, nitroglycerin is administered on 7 days a week.

Consequently, it is also envisaged in the context of the present invention that nitroglycerin is administered several times a day, but not on all days of the week, preferably on every other day.

According to the invention, nitroglycerin is administered during a defined time period in a gradually increasing amount. In the context of the present invention, it is to be understood that the term "defined time period" may vary dependent on the particular side effect to be reduced and, in addition, dependent on the patient to be treated.

In the context of the present invention, the defined time period lasts for 3 days to 40 weeks, preferably for 1 week to 16 weeks, more preferably for 3 to 4 weeks, and most preferred for at least two weeks.

Preferably, the defined time period lasts for two weeks.

Equally preferred is that the defined time period lasts for 3 days to one week.

Also preferred is that the defined time period lasts for 3 weeks.

It is to be understood that the defined time period may also last for 2 to 3 weeks or that any other lengths may be possible to define the time period according to the present invention in which an gradually increasing amount of nitroglycerin is administered to the subject in need thereof for reducing the side effects which go along with a treatment with nitroglycerin.

The length of the time period in which the amount of nitroglycerin is gradually increased as well as the individual administration schedule to prevent side effects may also be adjusted individually for each patient. In the context of short acting NO donators, for example, it is known that the level and duration of side effects strongly correlate with and/or depend on the individual sensitivity of the patient, on its bodyweight, physical condition, blood pressure etc.

Preferably, said defined time period is the initial phase of the treatment with nitroglycerin.

In a preferred embodiment, the initial phase lasts for two weeks.

In another preferred embodiment, the initial phase lasts for three or four weeks.

In yet another preferred embodiment, the initial phase lasts for three weeks.

Side effects of the treatment with a short acting NO donor are similar to the side effects of long acting NO donors, well-known in the art and include headache, dizziness, lightheadedness, syncope, vertigo, drowsiness, severe and prolonged headache, cerebral ischemia, faintness and somnolence.

Hence, in a preferred embodiment, the side effect to be reduced is selected from the group consisting of headache, dizziness, lightheadedness, syncope, vertigo, drowsiness, cerebral ischemia, faintness and somnolence.

More preferably, the side effect to be reduced is selected from the group consisting of headache, dizziness, lightheadedness, vertigo and drowsiness.

Most preferably, the side effect to be reduced is headache.

As the skilled person will appreciate, the administration of nitroglycerin may include an administration in one or more dosage forms, e.g. tablets or hubs (puffs). That is, nitroglycerin may be provided and administered in form of a spray or in form of a sublingual powder, preferably provided in sachets. For example, one administration may include the administration of two tablets or one to three hubs (puffs) or one to three sachets of sublingual powder, or any combination thereof.

Accordingly, in a preferred embodiment, nitroglycerin is administered in form of a powder, granules, a tablet, a capsule, a liquid, a gel, a solution or an aerosol.

Preferably, nitroglycerin is administered in form of a powder.

More preferably, nitroglycerin is administered in form of a powder, wherein the powder is in form of a sublingual powder. Even more preferably, the sublingual powder is provided in form of sachets or, alternatively, in form of stick packs.

According to the present invention, the initial phase is the phase in which dosages are administered in increasing amounts towards a predefined dosage (forced titration) or towards a dosage for optimal clinical treatment (individual titration). The initial phase should be as short as possible to reach the therapeutic dosage rapidly but long enough to prevent side effects and to allow sufficient patient adjustment. In the context of short acting NO donators, for example, it is known that the level and duration of side effects strongly correlate with and/or depend on the individual sensitivity of the patient, on its bodyweight, physical condition, etc. Therefore the optimal titration schedule could also be analyzed and monitored by a medical doctor or another skilled person.

However, it is also possible that the treatment with the NO donor has already started and has been stopped, e.g. due to the occurrence of side effects.

Hence, in another preferred embodiment, the defined time period is a phase which takes place during treatment with nitroglycerin, preferably after a first dosage of nitroglycerin has been administered which results in side effects.

In this case, the patient's treatment is restarted on the highest dose that is tolerated without side effects.

After a predefined or patient-optimized dosage is reached, the dosage is kept stable.

During this defined time period, according to the invention, the amount of nitroglycerin administered is gradually increased. Said increase may occur stepwise, i.e. in that the amount is increase once per week, followed by several administrations of the same amount. Alternatively, said increase may occur stepwise in that the amount is increased on a daily basis or after each administration until a final, a maximum concentration (or a plateau concentration) or therapeutic effective dose is reached. In a preferred embodiment, the initial amount of nitroglycerin which is administered during the defined time period is a subtherapeutical or less effective amount.

In the context of the present invention, the term "gradually increased" or "gradually increasing amount" means that the dosage which is administered to the patient is increased at least two times before the final and effective dosage for the treatment is administered. This can be achieved by increasing the dose for example every day, every other day, every third day, every week, fortnightly, every month, every other month or any other suitable time schedule. As the aim of a treatment is to achieve an effective therapeutic dosage as soon as possible and the aim of the intervention is the reduction of side effects, the skilled person would start with the highest dosage that is tolerated by the patient without side effects. The interval for the initial period may vary due to the nature of the side effects and to the constitution of the patient.

Preferably, a suitable initial dosage of nitroglycerin (GTN) for sensitive patients may be 0.15 mg of GTN with a biweekly increase of 0.15 mg of GTN, with the aim to reach a final and effective dosage of GTN of 0.45 mg which is then administered for, e.g., a time period of 16 weeks. The initial dose will be chosen to be lower if the patient complains about side effects.

Equally preferred is that the administration starts with a dosage of 0.15 mg of GTN, characterized in that this dosage is administered for a time period of two weeks. After the starting dosage has been administered for two week, the dosage is then, in a next step, increased to an amount of 0.3 mg of GTN. GTN is then administered for the next two weeks in an amount of 0.3 mg. In a second step, the dosage is increased to a final and effective dosage of 0.45 mg of GTN. In this example, the total treatment period lasts for 20 weeks, encompassing an initial period in which the dosage is gradually increased in two steps, starting with a dose of 0.15 mg of GTN for a time period of 2 weeks, followed by a dose of 0.3 mg for another 2 weeks and finally followed by a 16 week long treatment with the effective therapeutic dose of 0.45 mg of GTN.

In another preferred embodiment, the initial dosage may an amount of 0.3 mg of GTN with the dosage increasing gradually in two steps over a defined time period of three weeks to reach the optimal effective therapeutic dosage of 0.7 mg of GTN. The dosage is increased by 0.2 mg of GTN in every step. Every dosage is administered for at least one week, with the final therapeutic dose administered for at least one week, e.g. for a time period of two weeks, four weeks, ten weeks, 3 months, 6 months or any another suitable time interval.

Preferably, the drug administration starts with an initial dose of 0.3 mg nitroglycerin for one week, followed by a dose of 0.5 mg nitroglycerin for another week (titration step 1).

More preferably, the drug administration continues from week three on with a dose of 0.7 mg nitroglycerin (titration step 2) administered as a therapeutic dose for another ten weeks. Even more preferably, in this context, the nitroglycerin is administered to the patient at least three times a day.

In yet another preferred embodiment, the initial dosage may be 0.4 mg of GTN with a dosage increasing gradually in three steps over a defined time period of four weeks to reach the final and effective dosage of 1.2 mg of GTN. The starting dosage of 0.4 mg GTN could be administered for one week, the next dosage of 0.8 mg of GTN would then be administered for another (i.e. the second) week, the next dosage of 1.0 mg of GTN would be administered for another (i.e. the third) week. After three steps, the effective therapeutic active dosage of 1.2 mg GTN is finally reached in the fourth week.

Equally preferred is that the initial dosage of GTN may be administered in an amount of less than 0.15 mg of GTN, preferably in an amount which is in a range of 0.05 to 0.1 mg of GTN.

As mentioned above, the time period in which the starting dosage is gradually increased in at least two steps towards the therapeutically effective final dosage can vary significantly and also strongly depends on the individual response of the patient that is treated.

If the titration consists of at least two steps and the titration steps are relatively big, which means the following dosage is 100%, 130%, 140%, 150%, 200%, 250%, 300% or 400% of the previous, the duration of the individual titration step may be prolonged to allow optimal adjustment to the individual patient's response.

In one preferred embodiment of the invention, the defined time period is an ultrashort with a length of three days with a stepwise increase every day. Equally preferred is a time period of eight days with a stepwise increase every other day. Another preferred embodiment is in form of a time period of 12 days with a stepwise increase of dosage every third day. Yet another preferred embodiment is a time period of 3 weeks with a stepwise increase every week. Yet another preferred embodiment is a time period of six weeks with a stepwise increase of dosage every other week. If the patient is more sensible to the side effects yet another preferred embodiment of the invention is a long initial phase with a length of 9 weeks and a stepwise increase every third week.

Yet another preferred embodiment is a time period of 12 weeks with a stepwise increase every third to fourth week.

The length of the total titration period as well as the titration schedule may be varied due to the patient sensitivity and the individual patient's response.

The stepwise increase can be predefined in form of a fixed schedule (forced titration) or the increase can be triggered by a physiological response.

Preferably, at the onset of said defined time period, nitroglycerin is administered in a subtherapeutical or less effective amount. According to the invention, the term "subtherapeutical amount" refers to an amount which is not therapeutically effective.

According to the invention, a "less effective amount" represents an amount of the short acting NO donor which is already therapeutically active, but wherein the administered amount is not sufficient for triggering the desired therapeutic effect.

According to the invention, a "therapeutically active amount" is defined as an amount which results in the desired therapeutic effect, such as, for example, the intended physiological response.

Subtherapeutical as well as therapeutically active amounts of NO donors are known in the art. For example, with respect to nitroglycerin (GTN), the initial (and thereby subtherapeutical) amount may be in the concentration range of 0.05 mg, 0.1 mg, 0.2 mg or 0.3 mg, but are not limited thereto.

This amount may then be stepwise increased, e.g. within four weeks or within any of the periods as defined above, to 0.5, 0.6, 0.7, 0.8, 1.2 or 2.0 mg.

In a preferred embodiment of the invention, the final amount of nitroglycerin administered at the end of the defined time period is a therapeutically active amount.

Nitroglycerin can be administered in any suitable way so that it can be incorporated into the subject. This includes a parenteral or an intravenous administration as well as the administration to a mucous membrane of the subject.

Consequently, in a preferred embodiment of the present invention, nitroglycerin is administered lingually, sublingually, inhalatively, buccally, transmucosally or oromucosally.

Preferably, nitroglycerin is not swallowed.

Most preferably, nitroglycerin is administered parenterally.

Nitroglycerin can be formulated in any suitable way for the above mentioned administration modes.

In case of a lingual, sublingual or oromucosal administration, it is preferred that nitroglycerin is administered with the help of a spray, sprayable solution, a chewable capsule or in the form of a tablet, preferably a sublingual, buccal or chewable tablet, powder or granules or even by an inhalator device, from which nitroglycerin can be easily inhaled and adsorbed. It is equally preferred that nitroglycerin is administered in the form of an inhalable gas, aerosol or powder. Corresponding formulations are known to the person skilled in the art and include the formulation in a liquid in suitable buffers, in a gas, aerosol, as tablets, powder or granules.

Preferably, the administration of nitroglycerin is a non-topical administration, i.e., that nitroglycerin is not administered to the skin of the subject. In the context of the present invention, the term "skin" excludes mucous membranes of the subject.

Especially, the term "intermitting manner" means that nitroglycerin is formulated in a manner allowing an administration in an intermitting manner. Nitroglycerin is formulated in a way that allows a fast release of nitroglycerin from the formulation. Thereby, nitroglycerin may be released immediately (i.e. immediate-release formulation) as well as after a delayed period of time (delayed-release formulation) out of the preparation (e.g. formulation or dosage form) after having been administered to the subject. This includes e.g. formulations which do not hold back nitroglycerin for a longer time period and which are not characterized by a slow, sustained release, e.g. extended-release or retard preparations, but which release nitroglycerin within e.g., 30 or 15, 10, 5 minutes or 1 minute. The release may be also triggered upon an external signal, such as UV-light, temperature, ultrasound or a mechanical trigger.

According to the invention, nitroglycerin is used for the treatment or prevention of an arterial insufficiency.

According to the invention, the term "arterial insufficiency" refers to any insufficient blood or oxygen supply or any other insufficient supply of a tissue which is provided by an artery. This insufficient supply can be overcome by the present invention wherein nitroglycerin is used to increase the supply of a given tissue. The arterial insufficiency may occur both during physical rest and during an exercise.

In a preferred embodiment of the present invention, the arterial insufficiency is due to insufficient oxygen or blood supply of a tissue supplied by the artery or a bypass or shunt during physical rest or exercise.

According to a further preferred embodiment, the arterial insufficiency is due to an increased demand of oxygen or blood flow of a tissue supplied by the artery or a bypass or shunt.

This increased demand of oxygen or blood flow can have several reasons including but not limited to increased sport or physical activity, and increased mental activity or a disease requiring an increased demand of oxygen or blood flow.

According to a further preferred embodiment, the arterial insufficiency is characterized by a partial (stenosis) or complete occlusion of an arterial vessel. In the context of the present invention, the term "partial occlusion" is equivalent to a stenosis.

The partial or complete occlusion of an arterial vessel is a well-known phenomenon. It can have various reasons including, but not limited to, deposition of material in the blood vessels (including non-revascularisable stenoses), compression from external tissue or fluid next to the vessel (including disturbance in diastolic myocardial relaxation), vascular spasm, dysfunction of the endothelium of the vessel resulting in a paradoxic vasoconstriction during exercise or microvascular impairment due to endothelial dysfunction or smooth muscle cell abnormalities.

In a preferred embodiment, the arterial insufficiency is due to the deposition of material in the blood vessels.

The deposition of materials in the blood vessels is a well-known phenomenon resulting e.g. in atherosclerosis.

In a further preferred embodiment, the arterial insufficiency is due to an external or internal compression of an artery.

An internal compression of an artery may be due to an edema but also to a tumor putting pressure on the artery. Furthermore, this includes a vasospastical constriction of the artery as e.g. in Prinzmetal's angina. In addition, this also includes the paradoxic vasoconstriction which e.g. sometimes occur in an endothelial dysfunction or constricted small arterial vessels due to endothelial or smooth muscle cell dysfunction.

An external compression may be due to an accident or any external force which can put pressure on an artery.

In a further preferred embodiment, the arterial insufficiency is a vascular disease.

According to a further preferred embodiment, the arterial insufficiency is a disease selected from the group consisting of atherosclerosis, an ischemic disease and a further chronic arterial disease.

In a further preferred embodiment, the arterial insufficiency is a coronary arterial insufficiency.

In a preferred embodiment, the coronary insufficiency is an atherosclerotic coronary arterial insufficiency, in particular coronary artery disease (coronary heart disease or ischemic heart disease), stable angina pectoris, unstable angina pectoris, myocardial ischemia or chronic myocardial ischemia, acute coronary syndrome, or myocardial infarct (heart attack or ischemic myocardial infarct).

In a further preferred embodiment, the coronary insufficiency is a non-atherosclerotic, in particular coronary microvascular disease or small vessel disease, Prinzmetal's angina and cardiac syndrome X.

In a further preferred embodiment, the arterial insufficiency is a cerebral arterial insufficiency (intra- or extracranial).

In a preferred embodiment, the cerebral arterial insufficiency is an atherosclerotic cerebral arterial insufficiency, in particular cerebral ischemia, extracranial carotid artery disease, extracranial vertebral artery disease, pre-stroke, transient ischemic attack (mini stroke), stroke, vascular dementia, ischemic brain disease, or ischemic cerebrovascular disease.

The cerebral arterial insufficiency may also be ischemic microvascular brain disease, small vessel vascular dementia, subcortical arteriosclerotic encephalopathy (Binswanger's disease), Alzheimer's disease, or Parkinson's disease.

In a preferred embodiment, the arterial insufficiency is a peripheral arterial insufficiency.

In a preferred embodiment, the peripheral arterial insufficiency is an atherosclerotic peripheral arterial insufficiency, in particular peripheral vascular disease (peripheral artery disease (PAD) or peripheral artery occlusive disease (PAOD), including lower and upper extremity arterial disease).

In a preferred embodiment, the peripheral arterial insufficiency is an non-atherosclerotic peripheral arterial insufficiency, in particular Raynaud's syndrome (vasospasmatic), thrombangiitis obliterans, endangitis obliterans or Buerger's disease (recurring progressive inflammation and thrombosis (clotting) of small and medium arteries and veins of the hands and feet), vascular inflammatory disease (vasculitis), diabetic ischemia, diabetic neuropathy and compartment syndromes.

In a further preferred embodiment, the arterial insufficiency may be an intestinal arterial insufficiency, in particular an atherosclerotic intestinal arterial insufficiency, in particular ischemic bowel disease, mesenteric ischemia, or mesenteric infarction.

In a further preferred embodiment, the arterial insufficiency may be an urogenital arterial insufficiency, in particular an atherosclerotic urogenital arterial insufficiency, in particular erectile dysfunction, renal artery disease, renal ischemia, or renal infarction.

In a further preferred embodiment, the arterial insufficiency may be a nerval arterial insufficiency, in particular tinnitus.

Furthermore, the arterial insufficiency may be in the context of scleroderma (systemic sclerosis).

Furthermore, the arterial insufficiency may be in the context of fibromuscular dysplasia.

In a preferred embodiment, the arterial insufficiency is a central retinal artery insufficiency, in particular an atherosclerotic central retinal artery insufficiency, in particular ocular arterial insufficiency.

In a further preferred embodiment, the arterial insufficiency is characterized by an absence of an endothelial dysfunction.

The endothelial dysfunction is a well-known systemic pathological state of the endothelium and can be broadly defined as an imbalance between vasodilating and vasoconstricting substances produced by or acting on the endothelium.

In a further preferred embodiment, the arterial insufficiency is a chronic arterial insufficiency. In the context of the present invention, the term "chronic arterial insufficiency" means that the course of the arterial insufficiency is chronical and often progredient.

According to a further preferred embodiment, the chronic arterial insufficiency includes endothelial dysfunction, atherosclerosis, coronary artery disease (coronary heart disease or ischemic heart disease), stable angina pectoris, coronary microvascular disease or small vessel disease, Prinzmetal's angina and cardiac syndrome X, vascular dementia, ischemic brain disease, or ischemic cerebrovascular disease, ischemic microvascular brain disease, small vessel vascular dementia, subcortical atherosclerotic encephalopathy (Binswanger's disease), Alzheimer's disease, Parkinson's disease, peripheral vascular disease (peripheral artery disease (PAD) or peripheral artery occlusive disease (PAOD), thrombangiitis obliterans, endangitis obliterans or Buerger's disease, vascular inflammatory disease (vasculitis), fibromuscular dysplasia, diabetic ischemia, diabetic neuropathy, ischemic bowel disease, erectile dysfunction, renal artery disease, tinnitus, and scleroderma (systemic sclerosis).

In a further preferred embodiment, in case that nitroglycerin is used for the treatment or prevention of an arterial insufficiency, the short acting NO donor is administered in a final amount effective for the induction of arteriogenesis.

This implies that nitroglycerin can also be administered at various time points or, alternatively, for various time periods where there is no need for vasodilation and the relief of symptoms such as angina pectoris.

The skilled person will appreciate that this amount will depend on the patient in need of the treatment to which nitroglycerin is administered. Generally, the amount to be administered can vary due to the weight of the subject and its hemodynamic response to nitroglycerin. The daily amount of nitroglycerin (GTN) to be administered to a patient in need thereof may be in the range of between 0.1 mg and 10 mg of GTN, preferably in the range of between 0.5 mg and 5.0 mg of GTN.

In a preferred embodiment, treatment with nitroglycerin may be accompanied by physical exercise or by the application of an endogenous force to the arterial vessel.

According to the invention, the term "physical exercise" means any training of the subject, including but not limited to training in exercise rooms, jogging, walking (including walking on a tread mill), nordic walking, swimming, dancing, cycling and hiking. The skilled person will appreciate that any exercise will be helpful in the context of the invention, provided that it is performed in conjunction with the administration of nitroglycerin.

In a further aspect, the present invention also relates to nitroglycerin for use in a method for the prevention or treatment of an arterial insufficiency, wherein nitroglycerin is administered in an intermitting manner, and wherein during the initial phase of the administration, the amount of nitroglycerin is gradually increased at any dose from 0.05 mg to 2.0 mg, wherein the initial phase is 3 days to 40 weeks long.

All embodiments defined above with respect to the first aspect of the invention and relating to nitroglycerin, the administration in an intermitting manner, and the increase of the amount of nitroglycerin at the initial phase of the administration also apply to this aspect of the invention.

According to the invention, as to the total time of the treatment or prevention, it is possible to administer nitroglycerin for a period of several weeks or months.

In a preferred embodiment, nitroglycerin is administered for a time period of up to 8 to 10 weeks. It is equally preferred to administer the NO donor for 3 to 6, 3 to 8, 3 to 10, 4 to 8, 4 to 10, or 4 to 12 weeks. These time ranges are only examples and may vary depending on the individual schedule of the subject.

In a preferred embodiment, nitroglycerin is taken at least three times a week for at least 8 weeks, in particular for at least 12 weeks.

In another preferred embodiment, nitroglycerin is taken at least two to three times a day for 7 days a week and for at least 4 weeks, preferably for at least 8 weeks and more preferably for at least 12 weeks.

In a further preferred embodiment, nitroglycerin is taken not longer than 6, 8 or 12 months. However, it is also possible to take nitroglycerin for 2, 3 or even more years. Furthermore, it is also possible that the NO donor is administered for decades or even through the whole life of the subject.

In the context of such long term administrations, it is preferred that nitroglycerin is administered three or four times a week or at least three times a week.

Described, but not forming part of the present invention, is a method of promoting collateral circulation comprising the step of exposing a subject to a therapeutically effective amount of an NO donor, wherein the therapeutically effective amount of the NO donor promotes arteriogenesis sufficient to augment collateral circulation in a physiological or pathological condition, and wherein during the initial phase of the administration, the amount of said short acting NO donor is increased.

The term collateral circulation describes the circulation of blood through so-called collateral vessels. These vessels are small arterioles, which are part of a network that interconnects perfusion territories of arterial branches. In the case that the main artery itself is not capable of sufficiently supplying a tissue, e.g. due to an arterial occlusion, these collateral vessels are recruited and can develop to large conductance arteries, to bypass the site of an arterial occlusion and/or to compensate blood flow to ischemic territories supplied by the or insufficient artery. In the context of the present invention, the promotion of collateral circulation occurs via arteriogenesis.

According to the invention, the term "physiological condition" denotes any condition of the subject which is not related to any disease.

According to the invention, the term "pathological condition" denotes any condition of the subject which is related to a disease.

Preferably, the subject suffers from an arterial insufficiency.

All features and preferred embodiments discussed above for the method of treating or preventing an arterial insufficiency also apply to the method of promoting collateral circulation.

With respect to the aspects defined above where nitroglycerin is administered in a manner sufficient to induce arteriogenesis this manner is preferably an intermitting manner as defined above.

The invention is further explained by the following figures and examples, which are not intended to limit the present invention.

### FIGURE LEGENDS

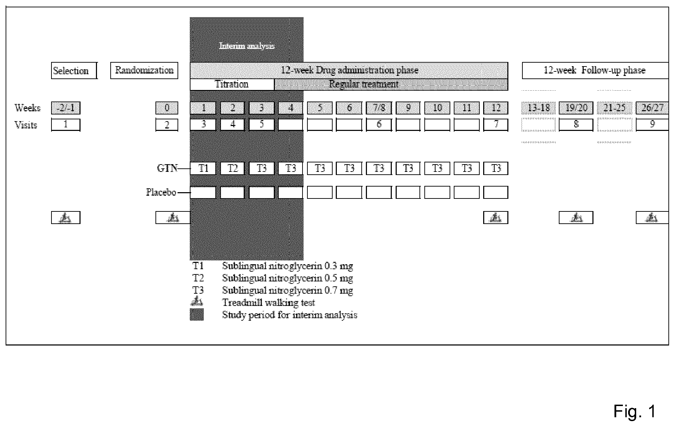

**Figure 1****:** Schematic representation of the multi-centre, randomized, placebo-controlled, double-blind trial to assess the efficacy and safety of nitroglycerin sublingual powder on the walking distance in a scheduled forced titration design in patients with peripheral artery occlusive disease (PAOD) and intermittent claudication (EUDRA-CT 2016-004460-19) (Example 3).

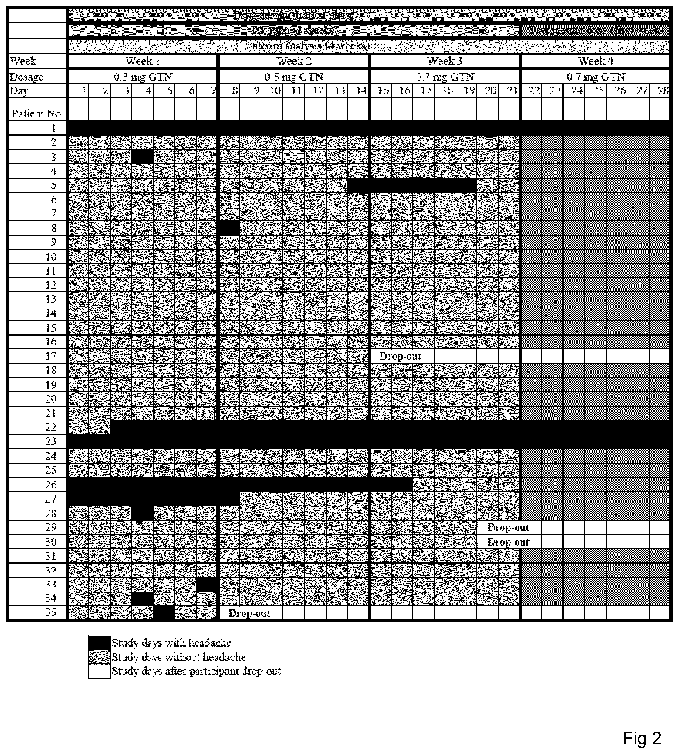

**Figure 2****:** Interim analysis of headache incidence in a multi-centre placebo-controlled, double-blind trial to assess the efficacy and safety of nitroglycerin sublingual powder on the walking distance in a scheduled forced titration design in patients with peripheral artery occlusive disease (PAOD) and intermittent claudication after finalized 3 week titration and first week of regular treatment (Example 3).

### EXAMPLES

### Example 1

A 35-year old men who is known to show severe headache when taking sublingual nitroglycerin in a therapeutic dose was put on a less effective amount of approximately 0,15 mg GTN TID (morning, noon, evening) sublingual powder to start. At this dosage he had no headache or other known GTN side effects. After a week he increased the dose to 0.3 mg GTN, after the second week to 0.5 mg, after another week he reached the final dosage of 0.7mg without side effects.

### Example 2

A 51-year old woman who responded with nausea and mild headache to an initial dose of 0.4 mg sublingual nitroglycerin was put on 0.15 mg TID (morning, noon, evening) to start. At this dosage she had no nausea or headache or other known GTN side effects. After a week, she increased the dose to 0.3 mg GTN TID, after the second week she reached the final dosage of 0.4 mg TID without side effects.

### Example 3

In a multi-centre, randomized, placebo-controlled, double-blind trial the efficacy and safety of nitroglycerin sublingual powder on the walking distance in a scheduled forced titration design in patients with peripheral arterial occlusive disease (PAOD) and intermittent claudication is assessed (EUDRA-CT 2016-004460-19). This clinical Phase Ila proof-of-concept study with 50 patients suffering from peripheral artery disease (PAD) and a pain free walking distance <200 metres (stadium Fontaine IIb) investigates the efficacy of an intermittent administration of nitroglycerin on the walking distance via the induction of arteriogenesis and consists of a 12-week phase of drug administration and a 12-week follow up phase without drug administration. In order to reduce the expected side effects of the short acting NO donor administration the study does not start with the therapeutic dose but follows a forced titration with two titration steps as described in the following. Drug administration starts with a low initial dose of 0.3 mg nitroglycerin for one week, followed by 0.5 mg (titration step 1) for another week. From the third week on the patients receive the final therapeutically active dosage of 0.7 mg nitroglycerin (titration step 2) for another ten weeks (Figure 1). The patients are advised to take the dosages in the morning, noon and evening so that the short acting NO donor is administered in an intermittent manner.

Study parameters are pain free walking distance (ICD= initial claudication distance) and maximum walking distance (ACD= absolute claudication distance) assessed by treadmill walking tests. These parameters are commonly accepted parameters in studies investigating the efficacy of therapies regarding peripheral artery disease. These parameters are the primary and secondary endpoints. Therefore for both parameters the increase in walking distance is measured. For the primary endpoint the difference from the walking distances of treadmill tests performed before the start of the drug administration phase (baseline) to week 12 (at the end of drug administration phase) is calculated.

For the secondary endpoint the difference of the walking distances of treadmill test performed before the start of the drug administration phase (baseline) to 6 weeks after the drug administration phase (first follow up visit at week 19-20) and to the end of the 12-week follow up phase (second follow up visit at week 26) is measured.

Additional secondary parameters are ankle-brachial index (ABI, difference from baseline to week 12, to week 19-20 and to week 26), questionnaires EQ-5D and ICQ (difference from baseline to week 12 and 19-20) and Oxygen saturation (SpO₂ before start of drug administration phase, at the start of the drug administration phase, at the end of the 12-week drug administration phase, 6 weeks after drugs administration phase and at the end of the 12-week follow up phase).

Participants report adverse effects during the study with headache being one of the adverse effects.

### Interim analysis of the ongoing study

The study has not yet been finalized but an interim analysis regarding headache as an adverse effect of the study drug has been performed (Figure 2, Table 1-3).

At the time point of the interim analysis the study was still blinded, so with regard to patients reported adverse event headache it could not be distinguished between the frequency of headache in the verum and the placebo group.

As the titration of the study medication takes place in the first 3 weeks, the analysis is focused on reported headache in the first three weeks plus in week 4 to also monitor possible late or ongoing side effects.

The analysis includes 35 participants so far which are at different time points of the study protocol. In Figure 1 the individual headache days of the 35 participants are shown. Some participants have already reached the end of the 12-week treatment period and are in the follow-up period, some participants are still on treatment, but all participants (33) have surpassed the titration phase and are on the final dosage of 0.7 mg GTN.

**Table 1: Number of participants and the number of weekly and total study days.**

| | |
|---|---|
| Number of participants | 35 |
| Number of study days per week | 245 (35x7) |
| Number of study days (week 1-4) | 980 (35x7x4) |
| Number of study days total (drug administration phase) | 2940 (35x7x12) |
| Number of drop-out days (drug administration phase) and not completed days of patients on treatment | 487 |
| Number of study days corrected by drop-out days (drug administration phase) and not completed days of patients on treatment | 2453 |
| Number of drop-out days (week 1-4 drug administration phase) | 53 |
| Number of study days corrected by drop-out days (week 1-4 drug administration phase) | 927 |

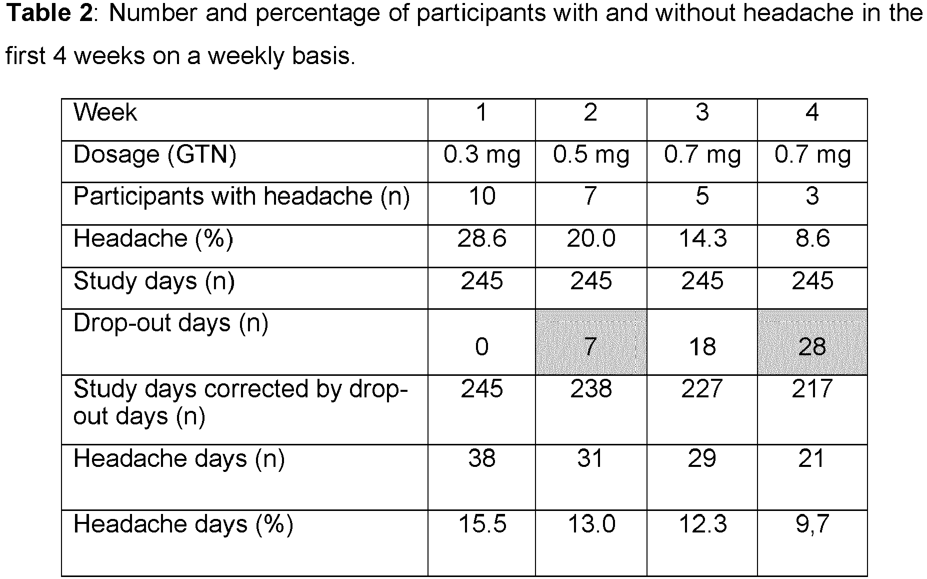

The number of participants with headache is subsequently reduced from n=10 in week 1 over n=7 in week 2 to n=5 in week 3 and n=3 in week 4.

Referring to the 35 participants this represents 28.6% of the participants in week 1. This initial percentage is reduced subsequently to 20% in the second week, 14.3% in the third and 8.6% in the fourth week.

Of the 35 participants 6 participants had headache on a single day in the first 4 weeks, but not during the first three days. 6 different participants had headache that lasted more than one day, in 5 of these the headache lasted more than a week.

Of the 927 study days (number of study days corrected by drop-out days in week 1-4 of the drug administration phase)during the first 4 weeks of the trial the participants had headache on 118 days which is an overall percentage of 12.7% with 15.5% in week 1, 13.0% in week 2, 12.3% in week3 and 9.7% in week 4.

245 participant headache days were recorded for a total of 2453 study days so far, which amounts to 10%.

209 headache days (85% of the total 245 headache days) were recorded for 3 participants that continuously had headache in the first 4 weeks and in the further course of the study.

There was one possibly headache related drop-out recorded during the titration in the first 4 weeks. In the further course of the therapy 2 additional participants dropped out due to headache.

### Conclusion

Due to the provisional character of the interim analysis it is not clear how an unblinding of the study data will influence the study outcome with regard to the side effect headache.

On basis of literature data an overall headache frequency of below 30% in the present study can be considered as low. During the therapy with short acting NO donors such as nitroglycerin or ISDN, headache is reported to be generally a very common side effect with a reported frequency of up to 64% when short acting NO donors are administered for therapeutical means.

In the present study it was found that the overall headache frequency started on a low 28.6 % in the first week and was subsequently reduced during the titration period to 8.6% in week 4. This is a low frequency in comparison to the reported rate of headache in the literature for sublingual nitroglycerin.

The percentage of headache as well as the intensity of headache during the therapy with nitrates is known to be dose-dependent. Therefore higher doses are expected to trigger a higher number of side effects. Without titration a dosage of 0.7 mg GTN is to be expected to generate more side effects than a dosage of 0.3 mg GTN.

In contrast thereto the present study did not monitor an increased headache frequency in week 2, 3 or 4 as a result of the increased dosage. Therefore the study results may confirm that the titration regime prevents a dose related increase of headache as a side effect, although it could not be distinguished between the frequency of headache in the verum and the placebo group due to the blinded data.

The adjustment process under a titration regime is expected to need some days, so that headaches in the first days of the overall titration period or the first days after each titration step might to be expected more frequent than in the following days or weeks. The titration starting dose (0.3 mg GTN) in this trial is significantly lower than the final therapeutic dosage (0.7 mg GTN) to smoothen the adjustment process. There are only 5 participants (14.3%) that have headache during the first days of treatment which is considered a lower percentage than expected without titration and a start with the therapeutical dose of 0.7 mg GTN.

### Limitations of the interim analysis and/or the trial in respect of the side effect headache

It is known, that irrespective of the type of clinical study and investigated drugs headache is a common side effect due to the high frequency in which spontaneous headache occurs in the population. In a study covering a period of twelve weeks spontaneous headache is to be expected besides drug-induced side effects. So, single headache days in the course of the study may be spontaneous headache and not necessarily drug-induced.

Therefore, it is anticipated that beside GTN-induced headache also spontaneous headache will be reported during the study in the verum as well as in the placebo group. This will probably influence the final number of drug-induced headaches.

In the present trial a history of chronic headache or migraine was no exclusion criterion. So there might be a fraction of the trial participants with a history of chronic headache or migraine. Chronic headache or migraine may have led to an overestimation of headache frequency as three participants had continuous headache during the complete study (resp. until drop-out).

3 participants with possible chronic headache or migraine background account for 65% of the headache days in the first 4 weeks (82 out of 127 headache days) and 85% of the headache days (212 out of 251 headache days) in the overall study.

**Table 3: Results with participants with possible chronic headache or migraine background excluded.**

| Week | 1 | 2 | 3 | 4 |
|---|---|---|---|---|
| Participants with Headache (n) | 10 | 7 | 5 | 3 |
| Participants with possible chronic headache or migraine history | 3 | 3 | 3 | 3 |
| Participants with headache corrected by participants with possible chronic headache or migraine history (n) | 7 | 4 | 2 | 0 |
| Headache corrected by participants with possible chronic headache or migraine history (%) | 20 | 11.4 | 5.7 | 0 |
| Participants headache days corrected by participants with possible chronic headache or migraine history (n) | 19 | 10 | 7 | 0 |
| Participant headache days corrected by participants with possible chronic headache or migraine history (%) | 7.8 | 4.2 | 3.1 | 0 |

When excluding the 3 trial participants due to their possible background of chronic headache or migraine from the data analysis the percentage of participants (and participant days) with headache is even lower. The remaining participants with headache would then amount to only 15% of participant headache days and only 20% of participants with headache in the first week. Titration reduces them to 11.4% in week 2 and 5.7% in week 3. In week 4 no participant (0%) (except the 3 suspected chronic headache or migraine participants) complained of headache as a side effect. This trend continued in weeks 5-12.

So far there has only been one participant drop-out likely due to headache in the titration phase, in 2 later drop-outs headache may have been the underlying cause. These dropped out participants may have a potential chronic headache or migraine background.

### Expected final study results

The study is planned to be finalized in 2019.

After the study is finalised and the data have been unblinded, we expect to find a lower frequency of headache in the titration verum group than reported for untitrated short acting nitrates use in literature data.

We expect the titration to reduce the side effect headache within the first three weeks to a low level or even prevent them completely despite the increasing dosage of nitroglycerin.

We also expect a low number of drop-outs during the beginning of the trial therapy, which is normally the most critical time in a trial as well as in real life therapeutic regimen.

## Claims

1. Nitroglycerin (GTN) for use in a method of reducing side effects of a treatment with nitroglycerin (GTN), wherein said treatment is for the treatment or prevention of an arterial insufficiency, comprising administering during a defined time period a gradually increasing amount of nitroglycerin (GTN) at any dose from 0.05 mg to 2.0 mg in an intermitting manner, wherein the defined time period is 3 days to 40 weeks long.

2. Nitroglycerin (GTN) for use in a method according to claim 1, wherein the defined time period is the initial phase of the treatment with nitroglycerin (GTN).

3. Nitroglycerin (GTN) for use in a method according to any one of claims 1 or 2, wherein said defined time period is 1 week to 16 weeks long, preferably 3 to 4 weeks long, and more preferably at least two weeks long.

4. Nitroglycerin (GTN) for use in a method according to any one of claims 1 to 3, wherein the initial amount of the nitrogylcerin (GTN) administered during the defined time period is a subtherapeutical or less effective amount.

5. Nitrogylcerin (GTN) for use in a method according to any one of claims 1 to 4, wherein the final amount of the nitrogylcerin (GTN) administered during the defined time period is a therapeutically active amount.

6. Nitroglycerin (GTN) for use in a method according to any one of claims 1 to 5, wherein the nitroglycerin (GTN) is administered at least once a day, preferably up to 2 to 3 times a day, more preferably up to 4 times a day.

7. Nitrogylcerin (GTN) for use in a method according to any one of claims 1 to 6, wherein the nitroglycerin (GTN) is administered lingually, sublingually, inhalatively, buccally, transmucosally or oromucosally.

8. Nitroglycerin (GTN) for use in a method according to claims 1 to 7, wherein the nitroglycerin (GTN) is administered in a final amount effective for the induction of arteriogenesis.

9. Nitroglycerin (GTN) for use in a method according to any one of claims 1 to 8, wherein the nitrogylcerin (GTN) is administered in form of a powder, granules, a tablet, a capsule, a liquid, a gel, a solution or an aerosol.

10. Nitroglycerin (GTN) for use in a method according to claim 9, wherein the NO donor is administered by means of a dosage form delivering a uniform dose, preferably in form of a stick pack, a tablet, a capsule, a (pre-)metered spray, a (pre-)metered inhaler, a (pre-)metered delivery device, or, alternatively, by means of a dosage form delivering an adjustable dose, preferably in form of an adjustable metering device.

11. Nitroglycerin (GTN) for use in a method for the prevention or treatment of an arterial insufficiency, wherein the nitroglycerin (GTN) is administered in an intermitting manner, and wherein during the initial phase of the administration, the amount of nitroglycerin (GTN) is gradually increased at any dose from 0.05 mg to 2.0 mg, wherein the initial phase is 3 days to 40 weeks long.

12. Nitroglycerin (GTN) for use in a method according to claim 11, wherein the initial phase is 1 week to 16 weeks long, preferably 3 to 4 weeks long, and more preferably at least two weeks long.

13. Nitroglycerin (GTN) for use in a method according to any one of claims 11 or 12, wherein the nitroglycerin (GTN) is defined as in any one of claims 2 to 10.

## Patentansprüche

1. Nitroglycerin (GTN) zur Verwendung in einem Verfahren zur Verringerung von Nebenwirkungen einer Behandlung mit Nitroglycerin (GTN), wobei die Behandlung zur Behandlung oder Vorbeugung einer arteriellen Insuffizienz dient, umfassend die Verabreichung einer graduell ansteigenden Menge von Nitroglycerin (GTN) bei einer beliebigen Dosis von 0,05 mg bis 2,0 mg in einer intermittierenden Weise während einer definierten Zeitperiode, wobei die definierte Zeitperiode 3 Tage bis 40 Wochen lang ist.

2. Nitroglycerin (GTN) zur Verwendung in einem Verfahren nach Anspruch 1, wobei die definierte Zeitperiode die Anfangsphase der Behandlung mit Nitroglycerin (GTN) ist.

3. Nitroglycerin (GTN) zur Verwendung in einem Verfahren nach einem beliebigen der Ansprüche 1 oder 2, wobei die definierte Zeitperiode 1 Woche bis 16 Wochen lang, vorzugsweise 3 bis 4 Wochen lang und besonders bevorzugt mindestens zwei Wochen lang ist.

4. Nitroglycerin (GTN) zur Verwendung in einem Verfahren nach einem beliebigen der Ansprüche 1 bis 3, wobei die anfängliche Menge des während der definierten Zeitperiode verabreichten Nitrogylcerins (GTN) eine subtherapeutische oder weniger wirksame Menge ist.

5. Nitrogylcerin (GTN) zur Verwendung in einem Verfahren nach einem beliebigen der Ansprüche 1 bis 4, wobei die Endmenge des während der definierten Zeitperiode verabreichten Nitrogylcerins (GTN) eine therapeutisch aktive Menge ist.

6. Nitroglycerin (GTN) zur Verwendung in einem Verfahren nach einem beliebigen der Ansprüche 1 bis 5, wobei das Nitroglycerin (GTN) mindestens einmal täglich, vorzugsweise bis zu 2- bis 3-mal täglich, besonders bevorzugt bis zu 4-mal täglich verabreicht wird.

7. Nitroglycerin (GTN) zur Verwendung in einem Verfahren nach einem beliebigen der Ansprüche 1 bis 6, wobei das Nitroglycerin (GTN) lingual, sublingual, inhalativ, bukkal, transmukosal oder oromukosal verabreicht wird.

8. Nitroglycerin (GTN) zur Verwendung in einem Verfahren nach einem der Ansprüche 1 bis 7, wobei das Nitroglycerin (GTN) in einer für die Induktion der Arteriogenese wirksamen Endmenge verabreicht wird.

9. Nitroglycerin (GTN) zur Verwendung in einem Verfahren nach einem beliebigen der Ansprüche 1 bis 8, wobei das Nitroglycerin (GTN) in Form eines Pulvers, Granulats, einer Tablette, einer Kapsel, einer Flüssigkeit, eines Gels, einer Lösung oder eines Aerosols verabreicht wird.

10. Nitroglycerin (GTN) zur Verwendung in einem Verfahren nach Anspruch 9, wobei der NO-Donor mittels einer Darreichungsform verabreicht wird, die eine gleichmäßige Dosis abgibt, vorzugsweise in Form einer Stick-Packung, einer Tablette, einer Kapsel, eines (vor-)dosierten Sprays, eines (vor-)dosierten Inhalators, einer (vordosierten Abgabevorrichtung oder alternativ mittels einer Darreichungsform, die eine einstellbare Dosis abgibt, vorzugsweise in Form einer einstellbaren Dosiervorrichtung.

11. Nitroglycerin (GTN) zur Verwendung in einem Verfahren zur Vorbeugung oder Behandlung einer arteriellen Insuffizienz, wobei das Nitroglycerin (GTN) in einer intermittierenden Weise verabreicht wird und wobei während der Anfangsphase der Verabreichung die Menge an Nitroglycerin (GTN) graduell bei einer beliebigen Dosis von 0,05 mg bis 2,0 mg erhöht wird, wobei die Anfangsphase 3 Tage bis 40 Wochen lang ist.

12. Nitroglycerin (GTN) zur Verwendung in einem Verfahren nach Anspruch 11, wobei die Anfangsphase 1 Woche bis 16 Wochen lang ist, vorzugsweise 3 bis 4 Wochen lang und besonders bevorzugt mindestens zwei Wochen lang.

13. Nitroglycerin (GTN) zur Verwendung in einem Verfahren nach einem beliebigen der Ansprüche 11 oder 12, wobei das Nitroglycerin (GTN) wie in einem beliebigen der Ansprüche 2 bis 10 definiert ist.

## Revendications

1. Nitroglycérine (GTN) pour une utilisation dans un procédé de réduction d'effets secondaires d'un traitement par nitroglycérine (GTN), dans laquelle ledit traitement est destiné au traitement ou à la prévention d'une insuffisance artérielle, comprenant l'administration pendant une période de temps définie d'une quantité progressivement croissante de nitroglycérine (GTN) à toute dose de 0,05 mg à 2,0 mg de manière intermittente, dans laquelle la période de temps définie dure de 3 jours à 40 semaines.

2. Nitroglycérine (GTN) pour une utilisation dans un procédé selon la revendication 1, dans laquelle la période de temps définie est la phase initiale du traitement par nitroglycérine (GTN).

3. Nitroglycérine (GTN) pour une utilisation dans un procédé selon l'une quelconque des revendications 1 ou 2, dans laquelle ladite période de temps définie dure 1 semaine à 16 semaines, de préférence 3 à 4 semaines, et de manière davantage préférée au moins deux semaines.

4. Nitroglycérine (GTN) pour une utilisation dans un procédé selon l'une quelconque des revendications 1 à 3, dans laquelle la quantité initiale de la nitroglycérine (GTN) administrée pendant la période de temps définie est une quantité sub-thérapeutique ou moins efficace.

5. Nitroglycérine (GTN) pour une utilisation dans un procédé selon l'une quelconque des revendications 1 à 4, dans laquelle la quantité finale de la nitroglycérine (GTN) administrée pendant la période de temps définie est une quantité thérapeutiquement active.

6. Nitroglycérine (GTN) pour une utilisation dans un procédé selon l'une quelconque des revendications 1 à 5, dans laquelle la nitroglycérine (GTN) est administrée au moins une fois par jour, de préférence jusqu'à 2 à 3 fois par jour, de manière davantage préférée jusqu'à 4 fois par jour.

7. Nitroglycérine (GTN) pour une utilisation dans un procédé selon l'une quelconque des revendications 1 à 6, dans laquelle la nitroglycérine (GTN) est administrée par voie linguale, sublinguale, par inhalation, par voie buccale, transmucosale ou oromucosale.

8. Nitroglycérine (GTN) pour une utilisation dans un procédé selon les revendications 1 à 7, dans laquelle la nitroglycérine (GTN) est administrée en une quantité finale efficace pour l'induction de l'artériogenèse.

9. Nitroglycérine (GTN) pour une utilisation dans un procédé selon l'une quelconque des revendications 1 à 8, dans laquelle la nitroglycérine (GTN) est administrée sous la forme d'une poudre, de granulés, d'un comprimé, d'une capsule, d'un liquide, d'un gel, d'une solution ou d'un aérosol.

10. Nitroglycérine (GTN) pour une utilisation dans un procédé selon la revendication 9, dans laquelle le donneur de NO est administré au moyen d'une forme posologique distribuant une dose uniforme, de préférence sous la forme d'un bâtonnet, d'un comprimé, d'une capsule, d'une pulvérisation (pré-) dosée, d'un inhalateur (pré-) dosé, d'un dispositif de distribution (pré-) dosé, ou, en variante, au moyen d'une forme posologique distribuant une dose réglable, de préférence sous la forme d'un dispositif de dosage réglable.

11. Nitroglycérine (GTN) pour une utilisation dans un procédé de prévention ou de traitement d'une insuffisance artérielle, dans laquelle la nitroglycérine (GTN) est administrée de manière intermittente, et dans laquelle pendant la phase initiale de l'administration, la quantité de nitroglycérine (GTN) est progressivement augmentée à toute dose de 0,05 mg à 2,0 mg, dans laquelle la phase initiale dure de 3 jours à 40 semaines.

12. Nitroglycérine (GTN) pour une utilisation dans un procédé selon la revendication 11, dans laquelle la phase initiale dure 1 semaine à 16 semaines, de préférence 3 à 4 semaines, et de manière davantage préférée au moins deux semaines.

13. Nitroglycérine (GTN) pour une utilisation dans un procédé selon l'une quelconque des revendications 11 ou 12, dans laquelle la nitroglycérine (GTN) est telle que définie dans l'une quelconque des revendications 2 à 10.
